# EUROPEAN PATENT SPECIFICATION

(11) **EP 0 698 385 B1**
(45) Date of publication and mention of the grant of the patent: **19.04.2000**
(21) Application number: 95870095.7
(22) Date of filing: 04.08.1995
(51) Int. Cl.: A61F 13/15, A61F 13/14

(54) **Breast-milk absorbent pad**
Saugkissen für Muttermilch
Coussin absorbant pour lait maternel

(30) Priority: 23.08.1994 JP 19862694
(43) Date of publication of application: 28.02.1996
(73) Proprietor: UNI-CHARM CORPORATION, Kawanoe-shi Ehime-ken (JP)
(72) Inventor: Murakami, Masaki, Kinsei-cho, Kawanoe-shi, Ehime-ken (JP); Shimizu, Shingo, Mitoyo-gun, Kagawa-ken (JP); Kato, Shinobu, Kanonji-shi, Kagawa-ken (JP)
(74) Representative: Van Malderen, Joelle

(56) References cited:
- DE-U- 9 305 155
- US-A- 3 442 268
- US-A- 5 149 336

## Description

The present invention relates to a breast-milk absorbent pad and, more particulary, to a breast-milk abosrbent pad used by women during their breast-feeding.

Conventional breast-milk absorbent pads generally comprise a liquid-permeable inner sheet, a liquid-impermeable outer sheet and a liquid-absorbent core formed of a mixture of fluff pulp fibers and superabsorbent polymer powders in a conic-shape.

However, such known breast-milk absorbent pads as mentioned above are inconvenient in that an attempt to obtain an agreeable touch for a woman's breasts would result in pads which are too soft to be kept in its conic-shape and to be easily inserted between a woman's brassieres and breasts. On the other hand, an attempt to facilitate the pads to be kept in the conic-shape would result in pads which are too hard to afford an agreeable touch.

As for a liquid absorption property of the breast-milk absorbent pad, a relatively soft pad is generally characterized by a density of fluff pulp fibers used for the core lower than that in a relatively hard pad and inferior to the hard pad in liquid diffusing as well as liquid holding properties in spite of its high instantaneous liquid absorption rate. Consequently, only a region of the core being in contact with a woman's nipples will be moistened but a peripheral region of the core remains dry and eventually the pad will be disposed although the pad still has an ability of liquid absorption in reserve. On the other hand, a relatively hard pad is generally characterized by a density of fluff pulp fibers higher than that of the soft pad and has a high liquid diffusing property but is inferior to the soft pad in the liquid absorption rate which is too low to overtake secretion of breast-milk if it is active.

The document US-A-3,442,268 is describing a pad for absorbing the lactal exudate from the female breast. Said pad is constructed with a central portion designed to be positioned over the nipple of the breast and an absorbent layer having portions of relatively high fluid transfer rate and portions of lower fluid transfer rate extending outwardly from the central portion. The portions of high fluid transfer rate are positioned so that they substantially surround the central portion and so that they are oriented in a substantially circumferential direction.

An objection of the invention is to solve the problems as described above by adjusting a fiber density in the core of the breast-milk absorbent pad so that the density may be lower in a central region than in a peripheral region of the core.

The object set forth above is achieved, according to the invention, by a breast-milk absorbent pad according to claim 1.

With the breast-milk absorbent pad of such construction, the feeling for a woman's nipples can be improved by decreasing the fiber density in the central region of the core to make this region softer than the peripheral region of the core while a shape stability of the pad can be improved by increasing the fiber density in the peripheral region to make this region harder than the central region.
Fig. 1 is a perspective view of a breast-milk absorbent pad according to the invention; and
Fig. 2 is a vertical sectional view of the breast-milk absorbent pad.

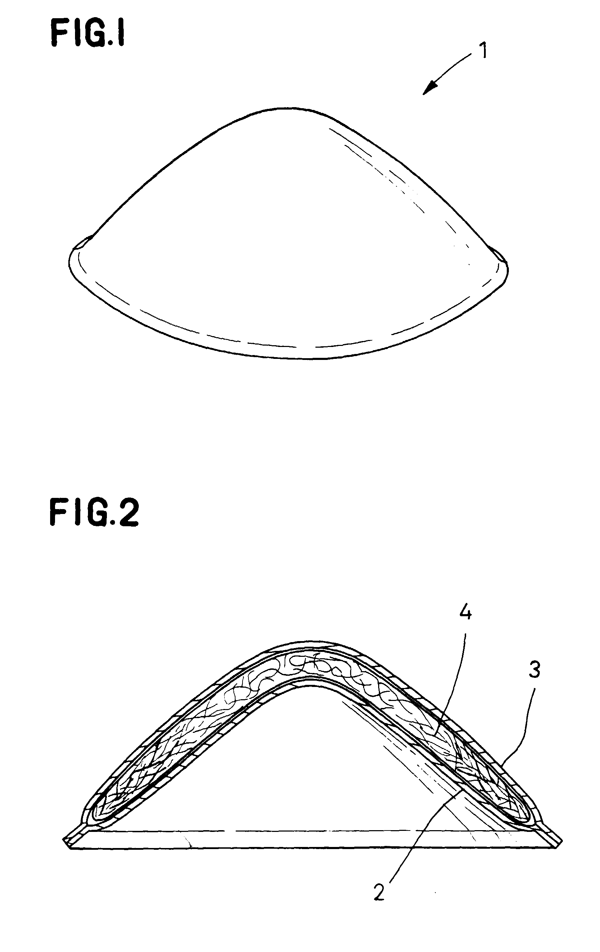

Referring to Figs. 1 and 2, a pad 1 comprises a liquid-permeable inner sheet 2, a liquid-impermeable outer sheet 3 and a liquid-absorbent core 4 disposed between these two sheets 2, 3, and is generally in a conic-shape having a height of 25 to 45mm and a diameter of 80 to 140mm. The inner and outer sheets 2, 3 are bonded to each other along portions thereof extending outward beyond a periphery of the core 4.

The inner sheet 2 is made of liquid-permeable material such as a woven or nonwoven fabric and the outer sheet 3 is made of liquid-impermeable material such as a plastic film or, if desired, a liquid-repellent treated woven or nonwoven fabric. The core 4 contains at least 30% by weight of hydrophilic fibers such as fluff pulp fibers or rayon fibers and a mixture of them and may additionally contain at most 20% by weight of thermoplastic synthetic fibers which have been subjected or not to a well known treatment making the thermoplastic synthetic fibers hydrophilic. Such synthetic fibers contribute to thermoformability, weldability and liquid diffusing property of the core. Furthermore, superabsorbent polymer powders may be uniformly mixed into the core 4 or may be spreaded over an intermediate plane of the core 4 in the direction of its thickness like a sandwich. At least the hydrophilic fibers of the fibrous materials used to form the core 4 is arranged so as to have a fiber density lower in the central region and higher in the peripheral region of the core 4, preferably, increasing and, more preferably, gradually increasing from the central region toward the opening peripheral edge of the core 4. For example, the fluff pulp fibers are uniformly distributed in the pad as a whole in a density of 145g/m², then compressed so that a density may be in a range of 0.1 to 0.15g/cm³ inside a central circular region defined by a radius of 15 to 25mm and gradually increase outwardly from the central circular region to a range of 0.25 to 0.30g/cm³ at the peripheral region, and so that a thickness of the core may also gradually increase outwardly from the central region toward the peripheral region. The term "fiber density" used in the embodiment means the number of individual fibers contained in a unit volume, but the fiber density in the present invention should not be restricted in this meaning.

With the pad 1 of such construction, the central region thereof is relatively soft and feels agreeable for a woman's nipple. On the other hand, the peripheral region is hard sufficiently to be kept in shape and facilitate the pad to be inserted between a woman's brassieres and the breasts. Secreted milk can rapidly permeate the pad from region of a lower fiber density toward the region of a higher fiber density, i.e., from the central region toward the peripheral region of the core 4. Therefore, the secreted breast-milk can spread all over the core and completely use it. In other words, the breast-milk absorbent pad 1 eliminates the wastefulness that the pad still having a milk absorbing capacity in reserve must be disposed.

The breast-milk absorbent pad according to the invention feels agreeable for a woman's nipple and is well kept in shape, since the fibrous material of the liquid-absorbent core has a gradient of fiber density lower in the central region and higher in the peripheral region of the pad. In addition, permeation of the secreted milk toward the peripheral region is sufficiently rapid to prevent the central region of the pad from being unacceptably moistened. This feature contributes to improve comfortableness to wear and allows the liquid absorptive materials of the core to be completely and effectively utilized.

## Claims

1. A breast-milk absorbent pad comprising:
- a liquid-permeable inner sheet;
- a liquid-impermeable outer sheet;
- a liquid-absorbent core disposed between said inner sheet and said outer sheet; and
- at least 30% by weight of said core comprising hydrophilic fibers so arranged to have a gradient of fiber density lower in a central region and higher in a peripheral region of said core,
characterised in that the fiber density of said core is increasing from the central region toward the peripheral region of said core.

2. A breast-milk absorbent pad according to claim 1, wherein said core is compressed so that said fiber density may gradually increasing from the central region toward the peripheral region of said core.

3. A breast-milk absorbent pad according to claim 1, wherein said core is compressed so that a thickness of said core may gradually decreasing from the central region toward the peripheral region of said core.

4. A breast-milk absorbent pad according to claim 1, wherein said core is harder in the peripheral region than in the central region of said core.

5. A breast-milk absorbent pad according to claim 1, wherein said core contains at most 20% by weight of thermoplastic synthetic fibers and is thermoformed.

6. A breast-milk absorbent pad according to claim 1, wherein said core is generally formed in a conic-shape.

## Patentansprüche

1. Brustmilchabsorbierendes Kissen, aufweisend:
- eine flüssigkeitsdurchlässige innere Schicht;
- eine flüssigkeitsundurchlässige äußere Schicht;
- einen flüssigkeitsabsorbierenden Kern, der zwischen der inneren Schicht und der äußeren Schicht angeordnet ist;
- wobei mindestens 30 Gewichtsprozent des Kerns hydrophile Fasern aufweisen, die so angeordnet sind, daß sie einen Faserdichtegradienten haben, der in einem zentralen Gebiet des Kerns niedriger ist und in einem peripheren Gebiet des Kerns höher ist,
dadurch gekennzeichnet, daß die Faserdichte des Kerns von dem zentralen Gebiet zu dem peripheren Gebiet des Kerns hin zunimmt.

2. Brustmilchabsorbierendes Kissen gemäß Anspruch 1, wobei der Kern so zusammengepreßt ist, daß die Faserdichte von dem zentralen Gebiet zu dem peripheren Gebiet des Kerns hin allmählich zunimmt.

3. Brustmilchabsorbierendes Kissen gemäß Anspruch 1, wobei der Kern so zusammengepreßt ist, daß die Dicke des Kerns von dem zentralen Gebiet zu dem peripheren Gebiet des Kerns hin allmählich abnimmt.

4. Brustmilchabsorbierendes Kissen gemäß Anspruch 1, wobei der Kern in dem peripheren Gebiet härter als in dem zentralen Gebiet des Kerns ist.

5. Brustmilchabsorbierendes Kissen gemäß Anspruch 1, wobei der Kern höchstens 20 Gewichtsprozent thermoplastische Synthesefasern enthält und thermogeformt ist.

6. Brustmilchabsorbierendes Kissen gemäß Anspruch 1, wobei der Kern im allgemeinen mit einer konischen Form geformt ist.

## Revendications

1. Compresse d'allaitement comprenant :
- une feuille interne perméable aux liquides,
- une feuille externe imperméable aux liquides,
- un noyau absorbant les liquides disposé entre ladite feuille interne et ladite feuille externe; et
- au moins 30% en poids dudit noyau comprenant des fibres hydrophiles agencées de manière à avoir un gradient de densité de fibres inférieur dans la région centrale et supérieur dans la région périphérique dudit noyau,
caractérisée en ce que la densité de fibres dudit noyau augmente de la région centrale vers la région périphérique dudit noyau.

2. Compresse d'allaitement selon la revendication 1, dans laquelle ledit noyau est comprimé de telle manière que ladite densité de fibres puisse augmenter graduellement de la région centrale vers la région périphérique dudit noyau.

3. Compresse d'allaitement selon la revendication 1, dans laquelle ledit noyau est comprimé de telle manière que l'épaisseur dudit noyau puisse diminuer graduellement de la région centrale vers la région périphérique dudit noyau.

4. Compresse d'allaitement selon la revendication 1, dans laquelle ledit noyau est plus dur dans la région périphérique que dans la région centrale dudit noyau.

5. Compresse d'allaitement selon la revendication 1, dans laquelle ledit noyau contient au maximum 20% en poids de fibres synthétiques thermoplastiques et est thermoformé.

6. Compresse d'allaitement selon la revendication 1, dans laquelle ledit noyau a généralement la forme d'un cône.
